# EUROPEAN PATENT APPLICATION

(11) **EP 2 857 389 A1**
(43) Date of publication of application: **08.04.2015**
(21) Application number: 14187756.3
(22) Date of filing: 06.10.2014
(51) Int. Cl.: C07D 277/40, A61K 31/426, A61P 3/10

(54) **CRYSTALLINE FORMS OF MIRABEGRON ACETATE SALT**

(30) Priority: 07.10.2013 IT MI20131653
(71) Applicant: Dipharma Francis S.r.l., 20021 Baranzate (MI) (IT)
(72) Inventor: Bove, Alessio, 20021 Baranzate (MI) (IT); Allegrini, Pietro, 20021 Baranzate (MI) (IT); Attolino, Emanuele, 20021 Baranzate (MI) (IT)
(74) Representative: Bertuccio, Silvia

(57) **Abstract**

The present invention relates to the acetate salt of Mirabegron, in particular in two novel crystalline forms, a process for their preparation and the use of said salt and its crystalline forms in the synthesis of Mirabegron with high yields and chemical purity.

## Description

The present invention relates to Mirabegron acetate salt, in particular in two novel crystalline forms, a process for their preparation, the use of said salt, in particular in crystalline form, as intermediate in the synthesis of Mirabegron with high yields and chemical purity, and a pharmaceutical composition containing said salt as active ingredient.

### PRIOR ART

(R)-2-(2-Aminothiazol-4-yl)-4'-[2-[(2-hydroxy-2-phenylethyl)amino]ethyl] acetanilide of formula **(I),** also known as Mirabegron, is a potent β-3 adrenergic agonist, which is primarily used in the treatment of urinary incontinence and disorders caused by overactive bladder in general.

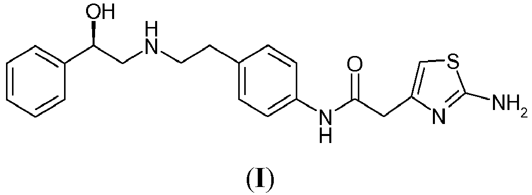

Mirabegron free base is known in two substantially anhydrous crystalline forms, designated as Forms **α** and **β**, described in US 7,342,117.

Crystalline form **α** of Mirabegron is a stable solid form, which is practically insoluble in water (its calculated solubility in water is only around 4 mg/l), whereas the crystal in form **β** is a metastable polymorph. Crystalline form **α** is the one currently used in slow-release Mirabegron tablets at doses of 25 and 50 mg.

Crystalline form **β** of Mirabegron was described in US 7,342,117 as being useful in the preparation of crystalline form α, which is commercially available. However, no specific technical advantage associated with the use of said crystalline form **β** as intermediate in the preparation of Mirabegron has been observed, in particular in the preparation of Mirabegron in crystalline form **α**.

Mirabegron as free base in amorphous solid form is also described in WO 2012/156998, and as dihydrochloride salt in crystalline form it is described in WO 99/20607.

As well known, the solubility of an active ingredient, and in particular of a crystalline or amorphous form thereof, significantly influences its bioavailability, and consequently its use for therapeutic purposes. In addition to solubility, the physical stability characteristics of a crystalline form of a pharmaceutical active ingredient also have a certain impact on its formulation use.

In particular US 7,342,117 demonstrates that Mirabegron, as dihydrochloride salt in crystalline form, is highly hygroscopic and unstable, and these characteristics make it unsuitable for formulation use.

There is therefore a need for a Mirabegron salt, which has optimum physicochemical characteristics for use in specific pharmaceutical formulations, and which can be useful in the preparation of Mirabegron, in particular of crystalline forms **α** or **β**, with high yields and purity.

### BRIEF DESCRIPTION OF FIGURES AND ANALYTIC METHODS

The different crystalline forms of Mirabegron acetate salt were characterised by the X-ray powder diffraction (XRPD) technique and by differential scanning calorimetry (DSC).

The X-ray powder diffraction (XRPD) spectra were collected with a Bruker D8-ADVANCE automatic powder diffractometer under the following operating conditions: geometry: Bragg-Brentano; generator: Radiation Cu Kα1 (λ= 1.54060 Å), Kα2 (λ= 1.54439 Å), Kβ (λ= 1.39222 Å); scanning: 2θ angle range of 3° to 40°, step size of 0.02° for 0.5 seconds. The detector is the energy-dispersive one-dimensional type (LinxEye XE).

The DSC thermograms for 2-(2-amino-1,3-thiazol-4-yl)-N-[4-(2-{[(2R)-2-hydroxy-2-phenylethyl]amino}ethyl)phenyl]acetamide acetate salt in the crystalline forms designated here as Forms γ and δ were acquired with the Mettler-Toledo DSC 822e differential scanning calorimeter under the following operating conditions: perforated aluminium capsule, range 30-250°C at the rate of 10°C/min, with nitrogen as purge gas (80 ml/min).

The water content of the crystalline forms of Mirabegron acetate salt was determined by titration with the Karl Fischer technique.

The particle size and D₅₀ of the crystalline forms of Mirabegron acetate salt were determined by the well-known laser light scattering technique, using a Malvern Mastersizer 3000 instrument.
Figure 1: XRPD diffractogram of solid in crystalline form γ showing the following main peaks expressed in 2θ: 8.85; 10.22; 12.47; 16.97; 17.71; 19.17; 19.77; 20.49; 22.36; and 22.87± 0.2°.
Figure 2: DSC thermogram of crystalline form γ of Mirabegron acetate salt wherein the endothermic peak at about 117°C indicates the fusion process.
Figure 3: XRPD diffractogram of solid in crystalline form δ showing the following main peaks expressed in 2θ: 10.65; 11.16; 12.36; 13.86; 14.14; 16.33; 16.62; 18.49; 20.70; 21.41; and 24.87± 0.2°.
Figure 4: DSC thermogram of crystalline form δ of Mirabegron acetate salt wherein the endothermic peak at about 119°C indicates the fusion process.

### SUMMARY OF THE INVENTION

The present invention relates to Mirabegron as acetate salt, in particular in crystalline form, more particularly in crystalline forms **γ** and **δ,** a process for their preparation and the use of said salt in the preparation of Mirabegron free base, in particular in crystalline forms **α** and **β**. The invention also provides a pharmaceutical composition containing Mirabegron as acetate salt, preferably in crystalline form, as active ingredient, and a pharmaceutically acceptable diluent and/or carrier.

### DETAILED DESCRIPTION OF THE INVENTION

The object of the present invention is Mirabegron acetate salt, preferably in crystalline form.

It has surprisingly been found that Mirabegron acetate salt presents numerous formulation advantages, mainly due to the physicochemical characteristics of crystalline forms **γ** and **δ** described below. Its use is also particularly advantageous in the preparation of Mirabegron free base, in particular with high yields and purity.

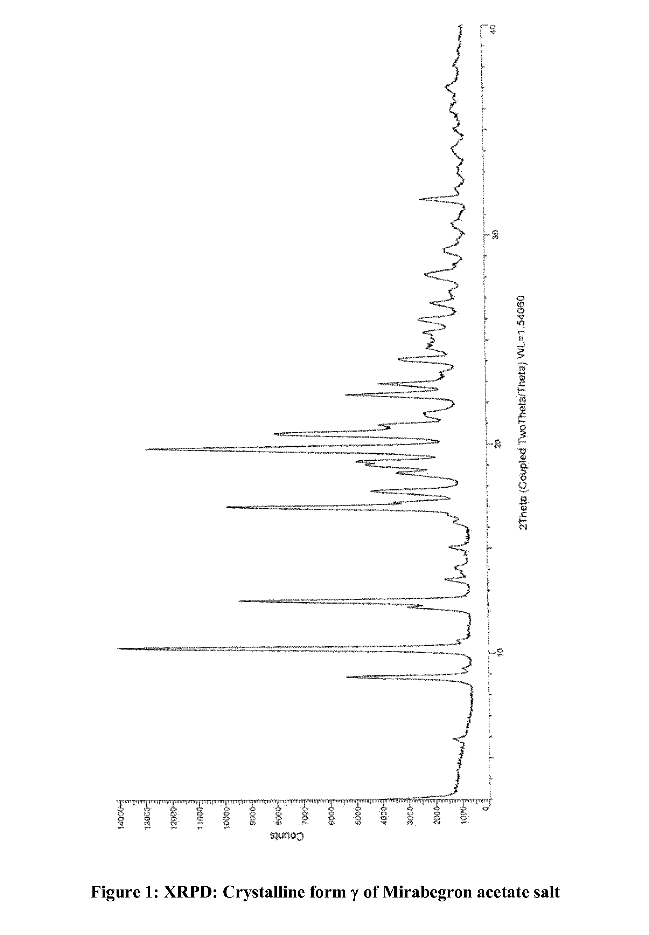

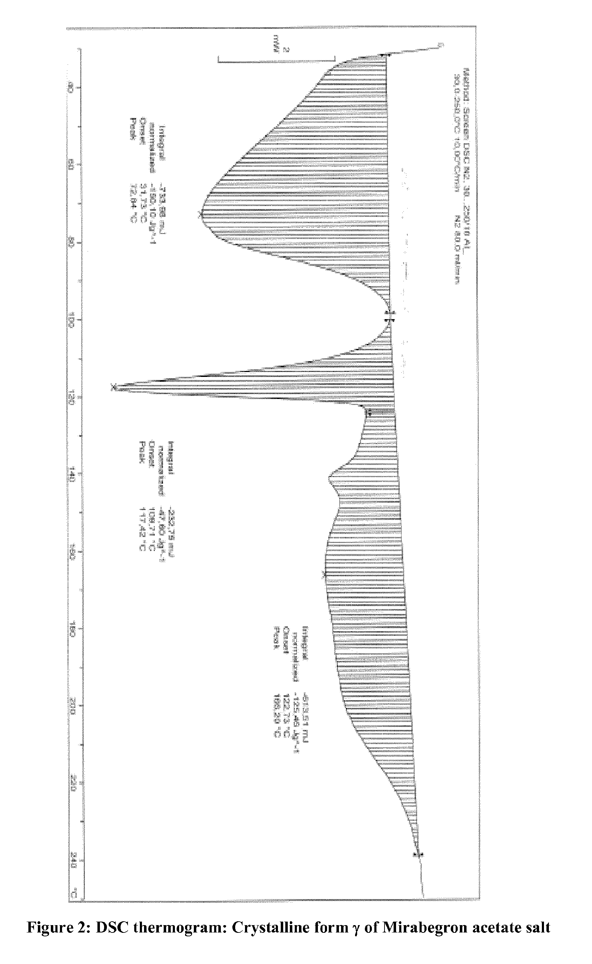

The invention also provides Mirabegron acetate salt in crystalline form, in particular in a crystalline form designated here as form **γ**, having a DSC pattern as substantially reported in Figure 2, wherein the main endotherm, which indicates the fusion process, is found at about 117°C, and an XRPD as substantially reported in Figure 1, wherein the characteristic peaks expressed in 2θ are found at about 8.85; 10.22; 12.47; 16.97; 17.71; 19.17; 19.77; 20.49; 22.36; and 22.87±0.2°.

Mirabegron acetate salt in crystalline form γ can be prepared by a process comprising:
a) formation of a solution or suspension of Mirabegron free base in a solvent;
b) addition of acetic acid or a salt thereof, soluble in the solvent referred to in step a), to the solution or suspension thus formed;
c) formation of a precipitate;
d) recovery of the crystalline solid.

Mirabegron free base of formula **(I),** as defined above, used as starting material, can be any known form of Mirabegron, such as a form of solid forms **α** or **β** described in US 7,342,117.

A solvent used in the formation of the solution or suspension of Mirabegon is typically selected from the group containing a straight or branched C₁-C₆ alkanol, such as a C₁-C₄ alkanol, for example methanol, ethanol, isopropanol or tert-butanol; a C₃-C₈ cyclic or acyclic aliphatic ketone, such as acetone, methyl ethyl ketone, methyl isobutyl ketone, 3-pentanone or cyclohexanone, preferably 3-pentanone; a cyclic or acyclic ether such as diethyl ether, diisopropyl ether, diisobutyl ether, tetrahydrofuran or dioxane, preferably tetrahydrofuran; a C₅-C₈ cyclic or acyclic aliphatic hydrocarbon such as pentane, hexane, heptane, cyclopentane, cyclohexane or cycloheptane; an aromatic hydrocarbon optionally substituted by a C₁-C₆ alkyl group or by one or more atoms of halogen, typically chlorine, such as toluene or chlorobenzene; a C₁-C₆ alkyl ester of a carboxylic acid, such as methyl acetate, ethyl acetate or propyl acetate; or a mixture of two or more, preferably two or three, of said solvents.

In a particularly preferred aspect of the invention the solvent used to form the solution or suspension referred to in step a) is selected from a C₃-C₈ ketone, in particular 3-pentanone, or an ether, in particular tetrahydrofuran.

The concentration of Mirabegron base in the starting solution or suspension can range between about 2 and 90% w/w, preferably between about 5 and 40%.

If necessary, to promote the formation of the solution of Mirabegron base of formula (I) referred to in step a), the mixture can be heated to a temperature ranging between about 20°C and the boiling point of the solvent, typically between about 25°C and 100°C, preferably about 50-90°C, until dissolution is complete.

Acetic acid, typically glacial acetic acid, or an acetic acid salt soluble in the solvent defined above in step a), typically a salt with a primary, secondary or tertiary amine, such as ammonium acetate, methyl ammonium, diethyl ammonium acetate, triethyl ammonium acetate and butyl ammonium acetate, is then added to the solution or suspension thus formed.

The molar ratio between the starting Mirabegron base and the acetic acid or a salt thereof is typically between about 1:1 and about 1:3, preferably around 1:1.

The acetic acid or salt thereof is added to the solution or suspension in a time ranging between about 5 minutes and 10 hours, preferably in about 5 minutes.

The precipitate can be formed by maintaining the solution or suspension under stirring, for example for a time ranging between about 10 minutes and about 20 hours, typically in about 2-8 hours.

If necessary, to promote the formation of the precipitate the solution can be cooled, for example to a temperature ranging between about -5° and 25°C, typically around about 20-25°.

Alternatively, to promote the formation of the precipitate, the solution or suspension can be seeded with a crystal of Mirabegron acetate in crystalline form **γ**, previously obtained.

The crystalline solid of Mirabegron acetate salt in crystalline form **γ** can be recovered by known techniques, such as filtration or centrifugation. In particular, if necessary, the recovery can be promoted by adding a suitable solvent to fluidify the dispersion, for example any solvent miscible with the solvent used to form the acetate salt, but wherein the acetate salt is not soluble.

The crystalline solid thus obtained can optionally be dried by known methods, for example in a stove at a temperature not exceeding about 20°C and 30°C, optionally under vacuum, to obtain a product having a water content lower than 1%.

The Mirabegron acetate solid in crystalline form thus obtained, in particular in the form designated here as Form **γ**, is an object of the invention.

It has now surprisingly been found that Mirabegron acetate in crystalline form **γ** can be converted to a further novel crystalline form of Mirabegron acetate salt, designated here as crystalline form **δ**.

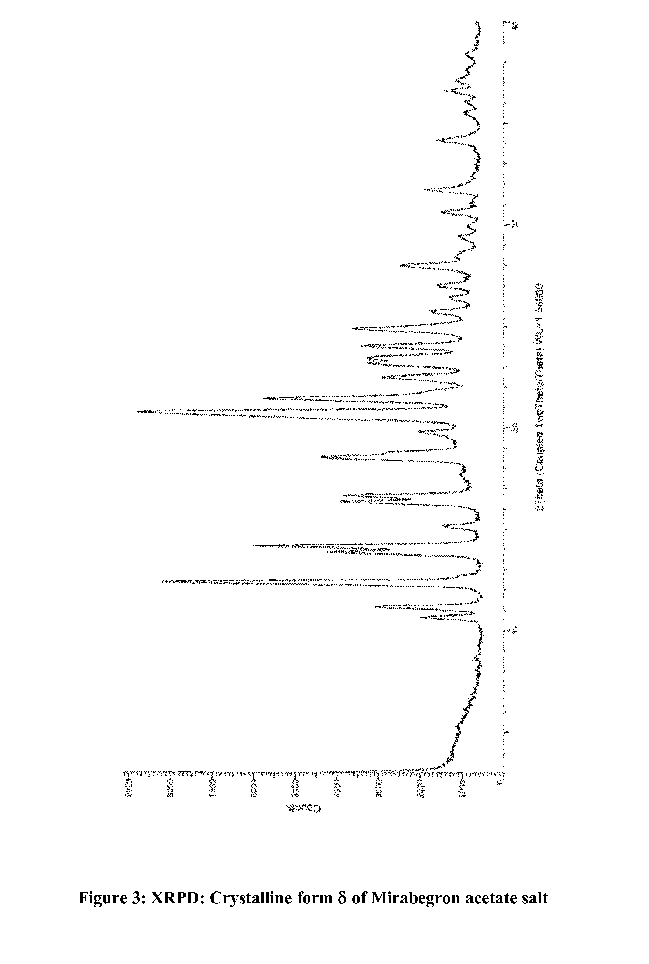

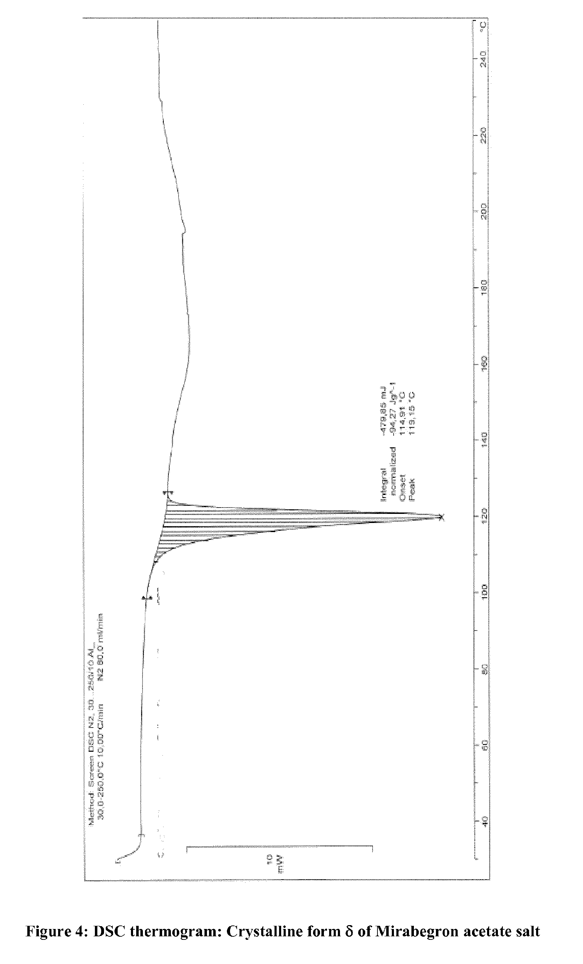

A further object of the invention is therefore a crystalline form of Mirabegron acetate designated here as form **δ,** having a water content lower than 1%, in particular around 0.3%, so that it can be defined as substantially anhydrous. Said form presents a DSC pattern substantially as reported in Figure 4, wherein the main endotherm, which indicates the fusion process, is found at about 119 °C, and an XRPD, as substantially reported in Figure 3, wherein the characteristic peaks expressed in 2θ are found at about 10.65; 11.16; 12.36; 13.86; 14.14; 16.33; 16.62; 18.49; 20.70; 21.41; and 24.87± 0.2°.

A further object of the invention is therefore a process for the preparation of Mirabegron acetate salt, in crystalline form **δ,** comprising heating Mirabegron acetate salt in crystalline form **γ**, preferably at low pressure, to a temperature ranging between about 30°C and about 70°C, preferably to about 50°C and 60°C. Said heating is typically performed in a time ranging between about 2 and 72 hours, preferably between about 10 and 30 hours.

The size of the crystals of Mirabegron acetate salt of formula (I) in crystalline form **γ** or **δ**, as obtainable by the processes described above, is characterised in both cases by a D₅₀ value ranging between about 25 and 250 µm. If desired, said values can be reduced by micronisation or fine grinding.

The Mirabegron acetate salt thus obtained in crystalline form **γ**, having a chemical purity calculated by HPLC exceeding 99.8%, can be converted to the corresponding Mirabegron acetate salt in crystalline form **δ** having the same chemical purity.

Mirabegron acetate salt in crystalline form **γ** and crystalline form **δ,** having a purity calculated by HPLC equal to or greater than 99.8%, is therefore a further object of the invention.

It has now surprisingly been found that crystalline forms of Mirabegron acetate salt **γ** and **δ,** as defined above, can be used as intermediates in the preparation of Mirabegron base, with high yields and purity, in particular in crystalline forms **α** and **β** known from US 7,342,117.

According to the prior art, Mirabegron base is obtainable with a chemical purity evaluated by HPLC at 99.4%, and its purification by crystallization to obtain Mirabegron base Form **α** produces a crystalline solid with an HPLC purity of 99.6%, which can be recrystallized to obtain a solid with an HPLC purity of 99.8%.

Surprisingly, it has been found that by subjecting the same Mirabegron base, having a chemical purity of 99.4% HPLC, to a purification process comprising the formation of Mirabegron acetate salt, the acetate salt can be obtained with a single crystallization with a purity, evaluated by HPLC, exceeding 99.8%, and therefore, following the release of the acetate salt, Mirabegron base, in solid form, in particular in crystalline form **α** and **β**, with an HPLC purity exceeding 99.8%.

A further object of the invention is therefore a process for the preparation of Mirabegron base in solid form, in particular in crystalline form **α** and **β**, comprising:
1) formation of a solution of Mirabegron acetate salt in a solvent selected from the group comprising water or a mixture thereof with a straight or branched C₁-C₆ alkanol;
2) addition of an organic or inorganic base;
3) precipitation of the solid; and
4) recovery of the solid.

The Mirabegron acetate used as starting material can be Mirabegron acetate, in particular in form **γ** or **δ,** or a mixture thereof, obtainable as previously described.

A straight or branched C₁-C₆ alkanol in step 1) is typically a C₁-C₄ alkanol, preferably methanol, ethanol, n-propanol, isopropanol, n-butanol, isobutanol or tert-butanol.

If desired, the formation of the solution referred to in point 1) can be facilitated by heating the mixture to a temperature ranging between about 20°C and 100°C, preferably about 25°C.

An organic base according to step 2) can be, for example, a C₁-C₄ alkoxide of an alkali metal, such as sodium or potassium; an inorganic base is typically selected from the group comprising an alkali metal hydroxide, an alkali metal carbonate or an alkali metal bicarbonate such as sodium or potassium. According to a preferred aspect of the invention the base is inorganic, and is sodium hydroxide.

The molar ratio between the organic or inorganic base and the starting Mirabegron acetate salt is typically between about 1:1 and about 3:1, preferably about 1:1.

Precipitation of the solid can be promoted by cooling, for example to a temperature ranging between about -5° and 25°C, typically about 20°C.

The Mirabegron base thus obtained can undergo a crystallization process to obtain, for example, its crystalline form **α** or **β**, according to known methods, such as those described in US 7,342,117.

In the precipitation of Mirabegron in step 3), the mixture can also be seeded by adding a seed of crystalline form of Mirabegron in form **α** or **β**, to obtain Mirabegron in crystalline form **α** or **β** respectively, for example as reported in US 7,342,117.

Mirabegron base, in particular in crystalline form **α** or **β**, can thus be recovered by known methods, typically filtration or centrifugation.

Alternatively, Mirabegron base, in particular in crystalline form **α** or **β**, can be obtained directly by a process comprising heating Mirabegron acetate salt, in particular in crystalline form γ or δ as defined above, to a temperature ranging between about 70°C and 110°C, preferably around 80-90°C.

Surprisingly, Mirabegron base, in particular in crystalline form **α**, **β**, **γ** or **δ,** obtained by the processes described above, has a purity, calculated by HPLC, equal to or greater than 99.8%, which meets the international regulatory requirements for active ingredients.

A further object of the invention is therefore a method of preparing Mirabegron base, in particular in crystalline form **α** or **β**, with a chemical purity equal to or greater than 99.8%, comprising the use as starting material of Mirabegron acetate salt, in particular in crystalline form **γ** or **δ** or a mixture thereof, having a purity exceeding 99.8%.

Unlike Mirabegron base, which is almost wholly insoluble in water, Mirabegron acetate salt has a solubility in water of almost 100 g/L at about 25°C.

A further object of the invention is a pharmaceutical composition comprising as active ingredient Mirabegron acetate salt, optionally in single crystalline form **γ** or **δ,** or a mixture thereof, and a pharmaceutically acceptable excipient and/or carrier and/or diluent.

Said pharmaceutical composition can be prepared in a pharmaceutical form by known methods. The dose of active ingredient Mirabegron acetate salt, optionally in crystalline form, for administration to humans, can be that commonly used in clinical practice for Mirabegron base, the selection of the most suitable dose being left to the clinician.

The following examples illustrate the invention.

### Example 1: Preparation of Mirabegron acetate salt in Form γ

A suspension of 2-(2-amino-1,3-thiazol-4-yl)-N-[4-(2-{[(2R)-2-hydroxy-2-phenylethyl]amino}ethyl)phenyl]acetamide (9 g, 23 mmol) in 3-pentanone (180 mL) is heated to 90°C. AcOH (1.4 g, 23 mmol) is added to the mixture in a time of about 5 minutes. The suspension is cooled in 6 hours to a temperature of 20°C. The crystallized solid is collected in a Büchner funnel and washed with 3-pentanone (20 mL). The solid is dried under nitrogen. The diffractogram of the solid in crystalline form γ presents an XRPD, as shown in Figure 1, which has the following most characteristic peaks expressed in 2θ: 8.85; 10.22; 12.47; 16.97; 17.71; 19.17; 19.77; 20.49; 22.36; and 22.87; and a DSC pattern as shown in Figure 2, wherein the main endotherm is found at about 117°C.

¹H NMR (DMSO-d₆, 300 MHz) δ (ppm): 9.97 (1H, s); 7.49 (2H, d J=8.4 Hz); 7.33-7.26 (4H, m); 7.23-7.21 (1H, m); 7.11 (2H, d J=8.4 Hz); 6.88 (2H, s); 6.29 (1H, s); 5.18 (1H, bs); 4.61-4.57 (1H, m); 3.45 (2H, s); 2.78-2.70 (2H, m); 2.65-2.58 (4H, m); 1.7 (1H, bs)

### Example 2: Preparation of Mirabegron acetate salt in Form δ

AcOH (0.47 g, 7.6 mmol) is added to a suspension of 2-(2-amino-1,3-thiazol-4-yl)-N-[4-(2-{[(2R)-2-hydroxy-2-phenylethyl]amino}ethyl)phenyl]acetamide (3 g, 7.6 mmol) in 3-pentanone (40 mL) at about 25°C. The mixture is maintained at the same temperature for 6 hours, and the solid precipitate is filtered through a Büchner funnel and washed with 3-pentanone (10 mL). The crystalline form of Mirabegron acetate thus obtained is crystalline form γ of Mirabegron acetate. Said crystalline form is then stove-heated at 50°C for 30 h at low pressure. The diffractogram of Mirabegron acetate in crystalline form δ has a DSC pattern as shown in Figure 4, which has the main endotherm at about 119°C, and presents an XRPD as shown in Figure 3, wherein the main peaks expressed in 2θ are found at: 10.65; 11.16; 12.36; 13.86; 14.14; 16.33; 16.62; 18.49; 20.70; 21.41; 24.87±0.2°.

### Example 3: Preparation of Mirabegron acetate salt in Form δ

The crystalline form γ of Mirabegron acetate obtained in example 1 is stove-heated at low pressure for about 30 hours at a temperature of about 50°C. The Mirabegron acetate in crystalline form **δ** thus obtained has a DSC pattern as shown in Figure 4, which has the main endotherm at about 119°C, and an XRPD as shown in Figure 3, wherein the most characteristic peaks are found at main peaks expressed in 2θ: 10.65; 11.16; 12.36; 13.86; 14.14; 16.33; 16.62; 18.49; 20.70; 21.41; and 24.87± 0.2°.

### Example 4: Preparation of Mirabegron acetate salt in Form γ

A suspension of 2-(2-amino-1,3-thiazol-4-yl)-N-[4-(2-{[(2R)-2-hydroxy-2-phenylethyl]amino{ethyl)phenyl]acetamide (HPLC purity 99.4%) (1.5 g; 3.8 mmol) in THF (25 mL) is heated to 50°C. AcOH (0.28 g; 3.8 mol) is added to the mixture. The mixture is cooled in 6 hours to a temperature of 25°C. The crystallised solid is collected in a Büchner funnel and washed with THF (10 mL). 1.5 g of 2-(2-amino-1,3-thiazol-4-yl)-N-[4-(2-{[(2R)-2-hydroxy-2-phenylethyl]amino}ethyl)phenyl]acetamide acetate salt with an HPLC purity exceeding 99.8% is obtained. Mirabegron acetate salt in crystalline form γ presents an XRPD, as shown in Figure 1, having the following most characteristic peaks expressed in 2θ: 8.85; 10.22; 12.47; 16.97; 17.71; 19.17; 19.77; 20.49; 22.36; and 22.87 and a DSC pattern as shown in Figure 2, wherein the main endotherm is found at about 117°C.

### Example 5: Formation of Mirabegron base of formula (I) from Mirabegron acetate salt

An aqueous solution of 30% NaOH (0.44 g ; 3.4 mol) is added to a solution of 2-(2-amino-1,3-thiazol-4-yl)-N-[4-(2-{[(2R)-2-hydroxy-2-phenylethyl]amino}ethyl)phenyl]acetamide acetate salt (1.7 g; 3.3 mmol) in H₂O (20 mL), maintaining the temperature at about 25°C. The solid precipitate is collected in a Büchner funnel and washed with H₂O (15 g). 1.1 g of 2-(2-amino-1,3-thiazol-4-yl)-N-[4-(2-{[(2R)-2-hydroxy-2-phenylethyl]amino}ethyl)phenyl]acetamide with an HPLC purity exceeding 99.8% is obtained.

### Example 6: Formation of Mirabegron base of formula (I) from Mirabegron acetate salt in crystalline form γ

Mirabegron acetate salt in crystalline form γ, obtained in accordance with Example 3, is stove-heated at a temperature of about 80°C for about 24 hours at low pressure to obtain Mirabegron with an estimated HPLC purity exceeding 99.8% and a quantitative yield of about 100%.

Proceeding as described in example 6, Mirabegron is obtained from Mirabegron acetate salt in gamma form.

## Claims

1. Mirabegron acetate salt, preferably in crystalline form.

2. Mirabegron acetate salt in crystalline form according to claim 1 selected from:
- Mirabegron acetate salt in crystalline form herein defined crystalline form **γ** having a DSC pattern wherein the main endothermic peak falls at about 117°C; and a XRPD wherein the most characterizing peaks in 2θ fall at about 8.85; 10.22; 12.47; 16.97; 17.71; 19.17; 19.77; 20.49; 22.36; and 22.87±0.2°; and
- Mirabegron acetate salt in crystalline form herein defined crystalline form **δ**, having a DSC pattern wherein the main endothermic peak falls at about 119°C; and a XRPD wherein the most characterizing peaks in 2θ fall at about 10.65; 11.16; 12.36; 13.86; 14.14; 16.33; 16.62; 18.49; 20.70; 21.41; and 24.87± 0.2°.

3. A process for the preparation of Mirabegron acetate salt in crystalline form **γ**, according to claim 2, comprising:
a) preparing a solution or suspension of Mirabegron free base in a solvent;
b) adding acetic acid or a salt thereof soluble in the solvent of step a) to solution or suspension of step a);
c) forming a precipitate; and
d) recovering the crystalline solid.

4. Process according to claim 3 wherein the solvent is selected from the group comprising a C₁-C₆ straight or branched alkanol; a C₃-C₈ cyclic or acyclic aliphatic ketone, preferably 3-pentanone; a cyclic or acyclic ether, preferably tetrahydrofuran; a C₅-C₈ cyclic or acyclic aliphatic hydrocarbon; an aromatic hydrocarbon optionally substituted with a C₁-C₆ alkyl group or with one or more halogen atoms; a C₁-C₆ alkyl ester of a carboxylic acid; or a mixture of two or more, preferably two or three, of said solvents.

5. A process according to claims 3 or 4 wherein the concentration of Mirabegron free base in the starting solution or suspension is comprised between about 2 and 90% w/w, preferably between about 5 and 40%.

6. A process according to claims 3-5 wherein the molar ratio between starting Mirabegron free base and acetic acid or a salt thereof is comprised between 1:1 and about 1:3, preferably between about 1:1.

7. A process for preparing Mirabegron acetate salt in crystalline form δ as defined in claim 2, comprising heating Mirabegron acetate salt as crystalline form γ, preferably under reduced pressure, at a temperature comprised between about 30°C and about 70°C, preferably at about 50°C-60°C.

8. A process according to claims 3-7 further comprising preparing Mirabegron free base in solid form, in particular in crystalline form α and β, by a process comprising:
1) forming a solution of Mirabegron acetate salt in a solvent selected from the group comprising water or a mixture thereof with a un C₁-C₆ straight or branched alkanol;
2) adding an organic or inorganic base;
3) precipitating the solid; and
4) recovering the solid; or
by a process comprising heating Mirabegron acetate salt, in particular in crystalline form γ or δ as defined in claim 2, at a temperature comprised between about 70°C and about 110°C, preferably at about 80-90°C.

9. Mirabegron acetate salt according to claims 1 or 2 having a purity calculated by HPLC equal to or greater than 99.8%; and/or a D₅₀ comprised between 25 and 250 µm.

10. A pharmaceutical composition comprising Mirabegron acetate salt, as active ingredient, in case in crystalline form **γ** or **δ,** or a mixture thereof, and a pharmaceutically acceptable excipient and/or carrier and/or diluent.
